# EUROPEAN PATENT SPECIFICATION

(11) **EP 3 177 360 B1**
(45) Date of publication and mention of the grant of the patent: **06.03.2024**
(21) Application number: 15750135.4
(22) Date of filing: 05.08.2015
(51) Int. Cl.: A61N 1/05, A61N 1/18, A61N 1/36

(54) **ELECTRODE LEADS FOR USE WITH IMPLANTABLE NEUROMUSCULAR ELECTRICAL STIMULATOR**
ELEKTRODENDRAHT ZUR VERWENDUNG MIT EINEM IMPLANTIERBAREN NEUROMUSKULÄREN ELEKTRISCHEN STIMULATOR
FILS D'ÉLECTRODES UTILISÉS AVEC UN STIMULATEUR ÉLECTRIQUE NEUROMUSCULAIRE IMPLANTABLE

(30) Priority: 06.08.2014 US 201414453423
(43) Date of publication of application: 14.06.2017
(73) Proprietor: Mainstay Medical Limited, Swords, County Dublin (IE)
(72) Inventor: SHIROFF, Jason Alan, Edina, Minnesota 55424 (US); SKUBITZ, Jason John, Arden Hills, Minnesota 55112 (US); RAWAT, Prashant Brijmohansingh, Blaine, Minnesota 55434 (US)
(74) Representative: Vossius & Partner Patentanwälte Rechtsanwälte mbB
(86) International application number: PCT/IB2015/055926
(87) International publication number: WO 2016/020846

(56) References cited:
- US-A- 5 897 584
- US-A1- 2007 060 967
- US-A1- 2008 103 570
- US-A1- 2008 103 574
- US-A1- 2012 089 153
- US-A1- 2012 192 874

## Description

### I. Field Of The Invention

This application generally relates to an apparatus for providing a lead for neuromuscular stimulation that is configured to limit axial forces and tensile load on a distal end of the lead and to reduce movement of the stimulation electrodes.

### II. Background Of The Invention

Many medical devices incorporate an elongated or tubular element that is required to be positioned at a particular anatomical site. Such devices include pacemakers, spinal cord and peripheral nerve stimulators for parathesia systems and functional electrical stimulation, and drug delivery catheters.

In the case of a pacemaker, for example, the leads may be threaded through a vein, and then anchored using a fixation element at the distal tip of the lead to reduce the risk of, or even prevent, dislodgement. Such a fixation element may be a tine, fin, or screw that is secured in the trabeculae or muscle tissue of the ventricle, atrium or cardiac vessel.

Sacral nerve stimulator leads may include a fixation element(s), such as a tine(s), projecting from the lead body to constrain movement of the lead body relative to the surrounding tissue. Tines on a sacral nerve lead, such as the InterStim^{™} lead available from Medtronic, Inc. of Fridley, Minnesota, generally are located at a substantial proximal distance from the electrodes and face in only one (proximal) direction. Such placement allows for relative movement of the electrodes as the muscle and connective tissue within which the tines are placed moves relative to the stimulation target.

A spinal cord stimulator (SCS) may include an implantable pulse generator (IPG) connected to one or more leads having one or more electrodes configured to deliver electrical energy to the spinal cord to block pain signals from reaching the brain. Small changes in electrode position may in some cases adversely impact the ability of such systems to effectively deliver therapy. It may not be practical or feasible to provide an anchoring mechanism inside the spinal canal to anchor a lead of the SCS. One conventional technique for securing the lead is to stabilize the lead using a ligature sleeve or suture sleeve secured to the lead body and attached to the superficial fascia with a suture as described, for example, in U.S. Patent No. 5,957,968 to Belden and U.S. Patent No. 7,930,039 to Olson. This technique, while commonly used, suffers from drawbacks including significant incidence of lead dislodgement. Another drawback is that the superficial tissue is often at an undesirable distance from the tissue targeted for stimulation. Any change in patient posture which results in a change in the relative distance between the superficial fascia and the stimulation target tissue may result in tension being applied to the lead body and subsequent movement of the electrodes.

U.S. Patent Nos. 8,428,728 and 8,606,358 to Sachs and U.S. Patent Application Publication No. 2011/0224665 to Crosby et al., all assigned to the assignee of the present invention, describe implanted electrical stimulation devices that are designed to restore neural drive and rehabilitate the multifidus muscle to improve stability of the spine. Rather than masking pain signals while the patient's spinal stability potentially undergoes further deterioration, the stimulator systems described in those applications are designed to reactivate the motor control system and/or strengthen the muscles that stabilize the spinal column, which in turn is expected to reduce persistent or recurrent pain. Sachs and Crosby also describe in alternative embodiments peripheral nerve stimulation, in which electrical energy is applied to a nerve to effect a physiological change, such as to elicit a muscle contraction.

While the stimulator systems described in the Sachs patents and Crosby application seek to rehabilitate the multifidus and restore neural drive, use of those systems necessitates the implantation of one or more electrode leads in the vicinity of a predetermined anatomical site, such as the medial branch of the dorsal ramus of the spinal nerve to elicit contraction of the lumbar multifidus muscle. For that application, there is no convenient anatomical structure near the distal end of the lead to allow use of conventional anchoring mechanisms. Anchoring the lead to the superficial fascia as described above initially may be effective in many cases, but leads anchored in this manner may be susceptible to the problems of dislodgement and fatigue-induced fracture.

Previously-known efforts to overcome the problems of lead displacement abound. For example, U.S. Patent No. 7,493,175 to Cates describes apparatus for subcutaneously anchoring a cardiac electrode lead using multiple tines. Such an apparatus would be undesirable for implantation in or adjacent to spinal muscle as the tines may become dislodged and tear the muscle during movement.

U.S. Patent No. 7,797,053 to Atkinson describes a tether and a stent like device at the distal portion of a lead that may be expanded inside a cardiac vein to anchor a cardiac pacing lead. A similar stent-like anchor for a neurostimulation lead is described in U.S. Patent No. 7,917,230 to Bly. U.S. Patent No. 7,908,015 to Lazeroms describes a stimulation lead to be placed subcutaneously in which the fixation mechanism includes a movable mechanism at the distal end of the lead such that the lead diameter is increased at the distal end when engaged to provide anchoring. U.S. Patent No. 8,170,690 to Morgan describes use of a helical element (screw) for anchoring a lead. These previously known anchoring systems are ill suited for neuromuscular stimulation because such systems have a high risk of dislodgement of the lead when implanted in or adjacent to muscle. US 2012/089153 A1 describes The present disclosure relates generally to an implantable stimulation system for stimulating and monitoring soft tissue in a patient, and more particularly, the present disclosure relates to systems and methods of using percutaneous delivery of a stimulation lead to treat sleep apnea and relates to various configurations of a stimulation electrode portion of a stimulation lead. US 2007/060967 A1 describes systems and methods that provide a stimulation system for prosthetic or therapeutic stimulation of muscles, nerves, or central nervous system tissue, or any combination. The stimulation systems and methods include an implantable pulse generator adapted for wireless telemetry and having a unique signature to enable secure communications. An external controller includes circuitry adapted for wireless telemetry. The secure communications includes at least a first communication from the external controller to the pulse generator. The first communication includes the pulse generator's unique signature. A response communication from the pulse generator to the external controller includes data elements that indicate the response communication is a response, and not a command from an external controller. US 2012/192874 A1 describes devices, systems and methods of neurostimulation for treating obstructive sleep apnea. US 2008/103570 A1 discloses a lead according to the preamble of claim 1.

It therefore would be desirable to provide electrode leads and methods of implantation wherein the lead is securely anchored within a patient and is able to absorb axial movement and tensile load without distributing the load to the distal anchored end, thus reducing the risk of dislodgement of the lead and/or lead fracture.

### III. Summary Of The Invention

The present invention overcomes the drawbacks of previously-known systems by providing a lead for neuromuscular electrical stimulation according to claim 1.

The strain relief portion may be a portion that is elastic and may include a helical coil conductor. The elastic strain relief portion also may include a sheath of insulative material having a lower durometer than the surrounding insulative sheath, thereby allowing the elastic portion to stretch more than the surrounding portions of the elongated member.

The conductor of the elongated member may be a coiled conductor or a cable conductor.

The strain relief portion may include the insulative sheath of the elongated member having a bellowed configuration and the at least one conductor comprising a coiled conductor. The strain relief portion alternatively or additionally may include the elongated member having a helical coiled configuration.

The strain relief portion includes a portion of the elongated member formed in a strain relief loop. The strain relief portion may be contained within a sealed pouch comprising a material which allows fluid ingression but reduces, or preferably prevents, tissue ingrowth.

### IV. Brief Description Of The Drawings

FIG. 1 shows an exemplary electrode lead having at least one distal fixation element and a strain relief portion.
FIG. 2 depicts the distal region of an exemplary electrode lead having a strain relief portion comprising a helical conductor portion on a proximal side of the electrodes.
FIG. 3 depicts the distal region of an exemplary electrode lead having a portion comprising lead material of a lower durometer than the surrounding lead body.
FIG. 4 shows the distal region of an exemplary electrode lead having a bellowed portion.
FIG. 5 shows the distal region of an exemplary electrode lead having a sigmoid portion.
FIG. 6 shows the distal region of an exemplary electrode lead having a helical portion.
FIG. 7 shows the distal region of an exemplary electrode having a strain relief loop formed therein and contained within a pouch (or elastomeric capsule), according to the invention.
FIGS. 8A and 8B depict, respectively, the distal region of an exemplary electrode having deployable fixation elements in a first insertion position where a distal tip is separated from a distal connection nut and in a second deployed position where a distal tip is coupled with a distal connection nut.
FIG. 9 is side sectional view of an exemplary distal tip having a threaded locking stylet inserted therein.
FIG. 10 is a side sectional view of an exemplary distal tip having a spring loaded locking element and a tapered locking stylet inserted therein.

### V. Detailed Description Of The Invention

The neuromuscular stimulation lead of the present invention comprises a lead body having a strain relief portion and a plurality of electrodes configured to provide electrical stimulation from an implantable pulse generator to neuromuscular tissue located within a patient's back. The leads disclosed herein are particularly adapted for use in stimulating tissue associated with the lumbar spine for use in restoring muscle function and lumbar spine stability, while overcoming lead displacement and fatigue fracture issues observed with previously-known electrode lead designs.

### Stimulation Lead with Strain Relief Portion

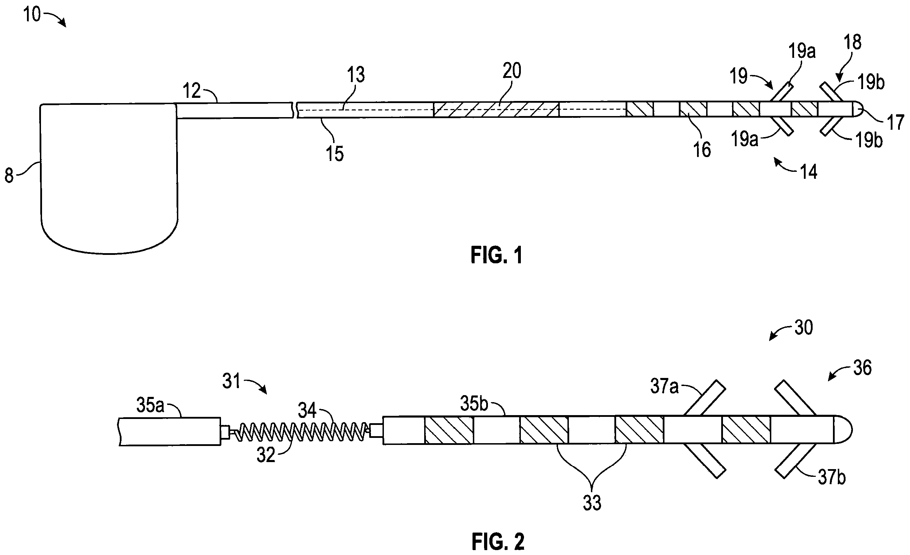

Referring to FIG. 1, exemplary stimulation lead 10 is described. Stimulation lead 10 includes proximal end 12, plurality of interior conductors 13, distal region 14, insulative sheath 15, electrodes 16, anchoring mechanism 18 including fixation elements 19a and 19b, and strain relief portion 20. Proximal end 12 of stimulation lead 10 is configured to be detachably attached to implantable pulse generator (IPG) 8 so that conductors 13 electrically couple IPG 8 to electrodes 16. Stimulation lead 10 illustratively has four electrodes 16, each coupled to a separate conductor 13 (only one shown), and are configured to be implanted in or adjacent to tissue, such as nervous tissue, muscle, ligament, and/or joint capsule.

Stimulation lead 10 is a suitable length for positioning electrodes 16 in or adjacent to target tissue while IPG 8 is implanted in a suitable location, e.g., the lower back. For example, stimulation lead 10 may be between about 30 and 80 cm in length, and preferably about 45 or about 65 cm in length. Stimulation lead 10 also has a diameter for placement within the muscles of the lumbar spine, for example, between about 1 and 2 mm in diameter and preferably about 1.3 mm.

Electrodes 16 may be configured to stimulate the tissue at a stimulation frequency and at a level and duration sufficient to cause muscle to contract and are ring electrodes. Electrodes 16 are a suitable length(s) and spaced apart a suitable distance along stimulation lead 10. For example, electrodes 16 may be about 2-5 mm in length, and preferably about 3 mm, and may be spaced apart about 2-6 mm, and preferably about 4 mm. As will also be understood by one of skill in the art, a stimulation lead may contain more or fewer than the four electrodes shown.

In the embodiment of FIG. 1, anchoring mechanism 18 includes fixation elements 19a and 19b, namely tines, which are configured to bracket an anchor site, e.g., muscle, therebetween to secure stimulation lead 10 at a target site without damaging the anchor site. Proximal fixation elements 19a are angled distally to resist motion in the distal direction and reduce the risk of over-insertion or migration of the lead in the distal direction. Distal fixation elements 19b are angled proximally and are configured to be deployed on the distal side of the tissue immediately adjacent to the target of stimulation. Fixation elements 19a and 19b accordingly reduce the risk of migration both proximally and distally.

The length of and spacing between the fixation elements is defined by the structure around which the fixation elements are to be placed. In one embodiment, the length of each fixation element is between about 1.5-4 mm and preferably about 2.5 mm and the spacing is between about 2 mm and 10 mm and preferably about 6 mm. Proximal and distal fixation elements 19a and 19b are configured to collapse inward toward stimulation lead 10 in a delivery state and to expand in a deployed state.

It was observed that during initial clinical testing involving the neuromuscular stimulation of the multifidus muscles of the lumbar spine with IPG 8 and conventional electrode leads, the leads frequently would dislodge and/or fracture after relatively short implantation periods. This was believed to be caused by the lack of suitable anchor sites for the stimulation leads, and also due to the torsional and bending stresses imposed on the stimulation leads by movement of the surrounding muscles. To address these issues, stimulation lead 10 therefore includes strain relief portion 20, which is configured to reduce axial strain on anchoring mechanism 18. In particular, as described below, strain relief portion 20 may take on a variety of structures that are designed to reduce the strain on stimulation lead 10 and anchoring mechanism 18, thereby reducing the risk of lead dislodgement, fatigue fracture, and injury to the tissue through which stimulation lead 10 passes. Each of the embodiments discussed below incorporates a strain relief portion 20, 31, 41, 51, 61, 71, 81 configured to be stretched or extended in response to axial displacements of the proximal part of the lead, and also to accommodate local flexion of, for example, the lumbar spine muscles that may cause localized lateral displacements of the stimulation lead.

Referring now to FIG. 2, stimulation lead 30 is described in which strain relief portion 31 comprises helical conductor 32. Helical conductor 32 preferably comprises a plurality of insulated wires that couple to the individual electrodes 33 and are enclosed within insulative sheath 34. Stimulation lead 30 further comprises lead body portions 35a and 35b, and anchoring mechanism 36, similar in design to anchoring mechanism 18 of FIG. 1. Anchoring mechanism 36 includes distally-directed tines 37a and proximally-directed tines 37b that are deployed, e.g., by proximally retracting a delivery sheath (not shown) during placement of the distal region of stimulation electrode 30.

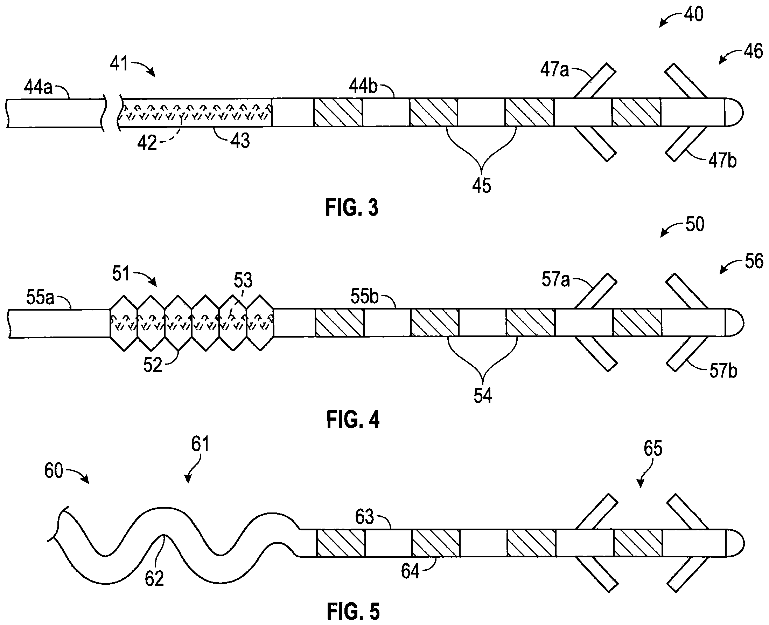

Referring to FIG. 3, stimulation lead 40 is constructed similarly to stimulation lead 30 of FIG. 2, except that strain relief portion 41 comprises helical conductor 42 (shown in dotted line) enclosed in a stretchable length of insulating tubing 43, so as to reduce, or preferably prevent tissue ingrowth that could reduce the elastic functionality of helical conductor 41. In particular, insulating tubing 43 may comprise a portion of tubing having lower durometer than the surrounding portions of tubing 44a and 44b. Accordingly, the combination of helical conductor 42 and portion of lower durometer tubing 43 may provide the elastic functionality of the strain relief portion. Other components of stimulation lead 40 include plurality of electrodes 45, and anchoring mechanism 46 comprising tines 47a and 47b, as discussed for the preceding embodiment.

FIG. 4 depicts an alternative of stimulation lead 50 having strain relief portion 51 comprising insulated tubing 52 having a bellows configuration. As for the embodiments of FIGS. 2 and 3, plurality of helical conductors 53 (one shown in dotted line) are provided to electrically couple electrodes 54 on body portion 55b to the proximal body portion 55a. As discussed above for the previous embodiments, stimulation lead 50 includes anchoring mechanism 56, preferably comprising deployable angled tines 57a and 57b.

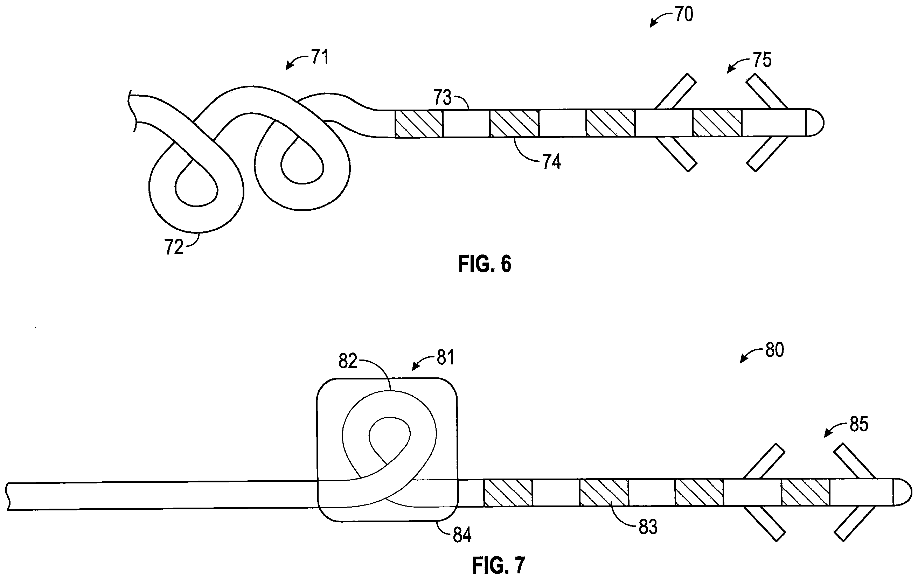

Referring now to FIGS. 5 and 6, further alternatives of stimulation leads including strain relief portions constructed in accordance with the present invention are described. In particular, FIG. 5 shows stimulation lead 60 having strain relief portion 61 comprising insulated tubing 62 formed in a sigmoid configuration that can be elastically stretched to a straightened form in response to the application of axial, lateral or torsional loads to stimulation lead 60. Other components of stimulation lead 60, including distal body portion 63, electrodes 64, and anchoring mechanism 65 may be constructed as described above for the preceding embodiments.

Similarly, the FIG. 6 embodiment depicts stimulation lead 70 having strain relief portion 71 comprising insulated tubing 72 formed in a coiled configuration that can be stretched in response to strains on the stimulation lead 70. Other components of stimulation lead 70, including distal body portion 73, electrodes 74, and anchoring mechanism 75 may be constructed as described above. Each of the stimulation leads in FIGS. 5 and 6 may include electrical conductors that match the shape of the sigmoid or coiled strain relief portion of the respective stimulation leads.
In FIG. 7, strain relief portion 81 of stimulation lead 80 comprises loop 82 of tubing containing electrical conductors that couple electrodes 83 to the proximal end of stimulation lead 80. Loop 82 is enclosed within sealed biocompatible elastomeric capsule 84. As with the preceding embodiments, loop 82 and capsule 84 permit extension of the stimulation lead between its proximal and distal ends without imposing excessive loads on anchoring mechanism 85 that could result in axial displacement of electrodes 83. In alternative embodiments, the capsule 84 may enclose a portion of the lead 80 having a sigmoid or helical coil configuration, as described above, or another configuration capable of extending in the axial direction. Elastomeric capsule preferably is watertight, but in some embodiments may permit fluid ingress so long as the capsule material reduces the opportunity for or prevents tissue ingrowth or tissue adhesion to the capsule that could limit the strain relief functionality of loop 82.

### Deployable Fixation Elements

Additional limitations to the effect of tensile loading on the distal end of a stimulation lead may be provided through additional support mechanisms for maintaining the fixation elements.

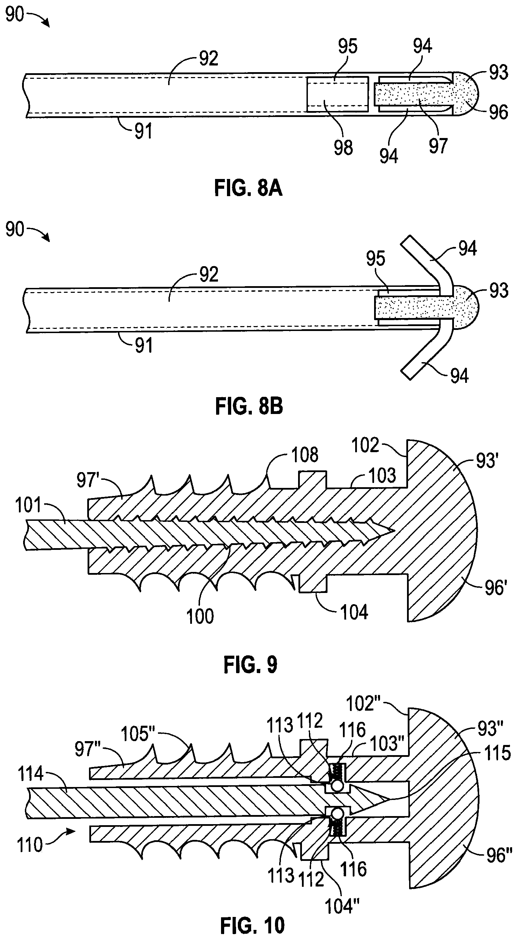

As shown in FIGS. 8A and 8B, another stimulation lead is provided. Stimulation lead 90 may include elongated body 91 having stylet lumen 92 extending therethrough, distal tip 93, expandable fixation elements 94, and nut 95. Stylet lumen 92 is shaped and sized to permit a stylet to be inserted therein, for example, during delivery of stimulation lead 90. Distal tip 93 has blunt head 96 configured to permit blunt dissection of tissue as lead 90 is inserted therethrough, and narrow body 97 sized for insertion in stylet lumen 92 such that blunt head 96 sealingly contacts the distal end of body 91. In one embodiment, distal tip 93 may be used to prevent the stylet from extending distally out of stylet lumen 92 beyond the distal end of the stimulation lead 90. Expandable fixation element 94 are configured to transition from a delivery state, shown in FIG. 8A, to a deployed state, shown in FIG. 8B. In the deployed state, expandable fixation elements 94 contact tissue and anchor lead 90 at a target location. Expandable fixation elements 94 are coupled to distal tip 93 and are sized to fit within stylet lumen 92 between narrow body 97 and body 91 in the deployed state while having a length suitable for anchoring in tissue in the deployed state. Nut 95 may be sized to fit within stylet lumen 92 and may be coupled to body 91 within lumen 92. Nut 95 includes lumen 98 sized to receive narrow body 97.

The embodiments of Figs. 8A and 8B provide for deploying fixation elements actively. FIG. 8A depicts the distal region of an exemplary stimulation lead having an expandable fixation element shown in a delivery state. Distal tip 93 is disposed at the distal end of elongated member 91. The proximal end of distal tip 93 interfaces with nut 95, also joined to stimulation lead 90, but more proximally. Between distal tip 93 and nut 95, stimulation lead 90 has longitudinal slits at each expandable fixation element 94 allowing elements 94 to move through the slits during deployment. Upon deployment of the electrode lead, depicted in FIG. 8B, distal tip 93 is driven proximally through nut lumen 98 within stimulation lead 90. As distal tip 93 moves proximally, the distal end of nut 95 contacts elements 94 and urges elements 94 to expand outwardly, as shown in FIG. 8B. Elements 94 may be located to provide stabilization within a tissue plane or between two adjacent tissue planes.

FIG. 9 illustrates an alternative distal tip 93' for use in stimulation lead 90 of FIGS. 8A and 8B, wherein like components are identified by like-primed reference numbers. Distal tip 93', includes ledge 102 of head 96', groove 103, ring 104, and coupling mechanism 105. Such features of distal tip 93' may also be present in the distal tip 93 of FIGS. 8A and 8B. Ledge 102 is configured to contact the distal end of the lead body. Groove 103 is configured to accept elements 94 for coupling. Ring 104 protrudes from narrow body 97' such that elements 94 are disposed in groove 103 between ring 104 and ledge 102. Narrow body 97' includes coupling mechanism 105, such as threads, ribs, or the like, for coupling distal tip 93' to nut 95. Alternative distal tip 93' further includes a threaded internal opening 100 configured to permit coupling to stylet 101 to provide axial strength for tensile loading during delivery and extraction by distributing forces over a large area of the fixation elements (and/or features of the lead and/or the lead itself) and to permit distal tip 93' to be moved proximally by pulling stylet 101 proximally. Such distribution of force is expected to reduce the risk of lead fracture during delivery and extraction.

Referring now to FIG. 10, another alternative distal tip 93" for use in stimulation lead 90 is provided. As will be observed by comparing FIGS. 9 and 10, distal tip 93" is similar to distal tip 93' and includes opening 110 rather than threaded opening 100, and locking groove 111, springs 112, and bearing 113. Opening 110 is configured to receive locking stylet 114 having tapered tip 115 and groove 116 proximal to tapered tip 115. Distal tip 93" has a spring loaded mechanism for locking onto tapered stylet 114, illustratively ball bearings 113 coupled to respective springs 112 which are disposed in grooves 111 in opening 110 of narrow body 97". Ball bearings 113 are biased inwardly by respective springs 112 which can be moved into groove 116 formed in tapered stylet 114 when stylet is inserted into opening 110, thereby locking stylet 114 in place. Groove 116 may be a single, bounded aperture in a portion of stylet 114, or may be a ridge formed about the full circumference of stylet 114. Stylet 114 is configured to provide axial strength for tensile loading during delivery and extraction by distributing forces over a large area of the fixation elements (and/or features of the lead and/or the lead itself) and to permit distal tip 93" to be moved proximally by pulling stylet 114 proximally to, for example, expand the expandable fixation elements.

Other locking stylets used for locking the distal tip into the position shown in FIGS. 8B and 10 may be used for supporting the expansion of the fixation elements and thereby supporting the position of the distal end of the stimulation lead at a desired stimulation site. In addition, as will be readily apparent to one of ordinary skill in the art, distal tips 93' and 93" may be used in embodiments of FIGS. 1-7 with stylets.

## Claims

1. A lead for neuromuscular electrical stimulation, the lead comprising:
an elongated member (10, 70, 80) having a proximal end (12), a distal region (14), a plurality of ring electrodes (16, 74, 83) and an anchoring mechanism (18, 75, 85) disposed on the distal region (14), and a plurality of electrical conductors (13) extending between the plurality of ring electrodes (16, 74, 83) and the proximal end (12);
a strain relief portion (20, 71, 81) comprising at least one loop (72, 82) of the elongated member (10, 70, 80) interposed between the proximal end (12) and the plurality of ring electrodes (16, 74, 83) and configured to reduce transmission of axial and lateral loads applied to the elongated member (10, 70, 80) to the distal region (14) and the anchoring mechanism (18, 75, 85); and
wherein the anchoring mechanism (18, 75, 85) comprises oppositely-directed deployable angled tines (19a, 19b) to secure the plurality of ring electrodes (16, 74, 83) in or adjacent to a desired anatomical site within a patient,
**characterized in that** the lead further comprises an elastomeric capsule (84) enclosing the at least one loop of the strain relief portion.

2. The lead of claim 1, wherein the strain relief portion (20, 71, 81) comprises a portion that is elastic.

3. The lead of claim 2, wherein the elastic portion (41) comprises at least one helical coil conductor (42).

4. The lead of claim 2, wherein the elastic portion (41) comprises a sheath of insulative material (43) having a lower durometer than the elongated member (40), thereby allowing the elastic portion (41) to stretch more than the elongated member (40).

5. The lead of any one of claims 1 to 4, wherein an electrode of the plurality of ring electrodes is disposed between the oppositely-directed deployable angled tines (19a, 19b).

6. The lead of claim 1, wherein the plurality of electrical conductors (13) comprises coiled portions (43).

7. The lead of any one of claims 1 to 6, wherein the plurality of electrical conductors (13) comprise extendable portions.

8. The lead of claim 7, wherein the elastomeric capsule (84) encloses the extendable portions.

9. The lead of any one of claims 1 to 8, wherein the oppositely-directed deployable angled tines (19a, 19b) comprise proximal fixation elements (19a) angled distally and distal fixation elements (19b) angled proximally, the proximal and distal fixation elements configured to bracket muscle at an anchor site to secure the lead at the anchor site without damaging the muscle.

10. The lead of any one of claims 1 to 9, wherein the lead comprises a distal tip (93) having a blunt tip (96) configured to permit blunt dissection of tissue as the lead is inserted therethrough.

## Patentansprüche

1. Draht für die neuromuskuläre elektrische Stimulation, wobei der Draht aufweist:
ein längliches Element (10, 70, 80) mit einem proximalen Ende (12), einem distalen Bereich (14), mehreren Ringelektroden (16, 74, 83) und einem Verankerungsmechanismus (18, 75, 85), der an dem distalen Bereich (14) angeordnet ist, und mehreren elektrischen Leitern (13), die sich zwischen den mehreren Ringelektroden (16, 74, 83) und dem proximalen Ende (12) erstrecken;
einen Zugentlastungsabschnitt (20, 71, 81), der mindestens eine Schleife (72, 82) des länglichen Elements (10, 70, 80) aufweist, die zwischen dem proximalen Ende (12) und den mehreren Ringelektroden (16, 74, 83) angeordnet und so konfiguriert ist, dass sie die Übertragung von axialen und lateralen Belastungen, die auf das längliche Element (10, 70, 80) ausgeübt werden, auf den distalen Bereich (14) und den Verankerungsmechanismus (18, 75, 85) verringert; und
wobei der Verankerungsmechanismus (18, 75, 85) entgegengesetzt gerichtete, entfaltbare, abgewinkelte Zinken (19a, 19b) aufweist, um die mehreren Ringelektroden (16, 74, 83) in oder neben einer gewünschten anatomischen Stelle innerhalb eines Patienten zu befestigen,
**dadurch gekennzeichnet, dass** der Draht ferner eine Elastomerkapsel (84) aufweist, die die mindestens eine Schleife des Zugentlastungsabschnitts umschließt.

2. Draht nach Anspruch 1, wobei der Zugentlastungsabschnitt (20, 71, 81) einen Abschnitt aufweist, der elastisch ist.

3. Draht nach Anspruch 2, wobei der elastische Abschnitt (41) mindestens einen spiralförmigen Spulenleiter (42) aufweist.

4. Draht nach Anspruch 2, wobei der elastische Abschnitt (41) eine Ummantelung aus isolierendem Material (43) mit einem niedrigeren Durometer als das längliche Element (40) aufweist, wodurch sich der elastische Abschnitt (41) stärker dehnen kann als das längliche Element (40).

5. Draht nach einem der Ansprüche 1 bis 4, **dadurch gekennzeichnet, dass** eine Elektrode der mehreren Ringelektroden zwischen den entgegengesetzt gerichteten, entfaltbaren, abgewinkelten Zinken (19a, 19b) angeordnet ist.

6. Draht nach Anspruch 1, wobei die mehreren elektrischen Leiter (13) gewickelte Abschnitte (43) aufweisen.

7. Draht nach einem der Ansprüche 1 bis 6, wobei die mehreren elektrischen Leiter (13) ausziehbare Abschnitte aufweisen.

8. Draht nach Anspruch 7, wobei die Elastomerkapsel (84) die ausziehbaren Abschnitte umschließt.

9. Draht nach einem der Ansprüche 1 bis 8, wobei die entgegengesetzt gerichteten, entfaltbaren, abgewinkelten Zinken (19a, 19b) proximale Befestigungselemente (19a), die distal abgewinkelt sind, und distale Befestigungselemente (19b), die proximal abgewinkelt sind, aufweisen, wobei die proximalen und distalen Befestigungselemente so konfiguriert sind, dass sie den Muskel an einer Verankerungsstelle umklammern, um den Draht an der Verankerungsstelle zu sichern, ohne den Muskel zu beschädigen.

10. Draht nach einem der Ansprüche 1 bis 9, wobei der Draht eine distale Spitze (93) mit einer stumpfen Spitze (96) aufweist, die so konfiguriert ist, dass sie eine stumpfe Dissektion von Gewebe ermöglicht, wenn der Draht durch sie hindurch eingeführt wird.

## Revendications

1. Câble pour stimulation électrique neuromusculaire, ledit câble comprenant :
un élément allongé (10, 70, 80) ayant une extrémité proximale (12), une partie distale (14), une pluralité d'électrodes annulaires (16, 74, 83) et un mécanisme d'ancrage (18, 75, 85) disposé sur la partie distale (14), et une pluralité de conducteurs électriques (13) s'étendant entre la pluralité d'électrodes annulaires (16, 74, 83) et l'extrémité proximale (12) ;
une partie de décharge de traction (20, 71, 81) comprenant au moins une boucle (72, 82) de l'élément allongé (10, 70, 80) intercalée entre l'extrémité proximale (12) et la pluralité d'électrodes annulaires (16, 74, 83) et prévue pour réduire la transmission vers la partie distale (14) et le mécanisme d'ancrage (18, 75, 85) des charges axiales et latérales appliquées à l'élément allongé (10, 70, 80) ; et
où le mécanisme d'ancrage (18, 75, 85) comprend des dents inclinées déployables orientées dans des directions opposées (19a, 19b) pour fixer la pluralité d'électrodes annulaires (16, 74, 83) sur un site anatomique souhaité d'un patient ou à côté de celui-ci, **caractérisé en ce que** ledit câble comprend en outre une capsule élastomère (84) renfermant ladite au moins une boucle de la partie de décharge de traction.

2. Câble selon la revendication 1, où la partie de décharge de traction (20, 71, 81) comprend une partie élastique.

3. Câble selon la revendication 2, où la partie élastique (41) comprend au moins un conducteur à enroulement hélicoïdal (42).

4. Câble selon la revendication 2, où la partie élastique (41) comprend une gaine de matériau isolant (43) ayant un duromètre inférieur à celui de l'élément allongé (40), permettant ainsi à la partie élastique (41) de s'étirer davantage que l'élément allongé (40).

5. Câble selon l'une des revendications 1 à 4, où une électrode de la pluralité d'électrodes annulaires est disposée entre les dents inclinées déployables orientées dans des directions opposées (19a, 19b).

6. Câble selon la revendication 1, où la pluralité de conducteurs électriques (13) comprend des parties enroulées (43).

7. Câble selon l'une des revendications 1 à 6, où la pluralité de conducteurs électriques (13) comprend des parties extensibles.

8. Câble selon la revendication 7, où la capsule élastomère (84) contient les parties extensibles.

9. Câble selon l'une des revendications 1 à 8, où les dents angulaires déployables orientées dans des directions opposées (19a, 19b) comprennent des éléments de fixation proximale (19a) inclinés distalement et des éléments de fixation distale (19b) inclinés proximalement, lesdits éléments de fixation proximale et distale étant prévus pour enserrer un muscle sur un site d'ancrage afin de fixer le câble au site d'ancrage sans endommager le muscle.

10. Câble selon l'une des revendications 1 à 9, où ledit câble comprend une extrémité distale (93) dotée d'une pointe émoussée (96) prévue pour permettre une dissection émoussée des tissus lorsque le câble est inséré dans ceux-ci.
